# EUROPEAN PATENT SPECIFICATION

(11) **EP 3 971 217 B1**
(45) Date of publication and mention of the grant of the patent: **27.12.2023**
(21) Application number: 20805799.2
(22) Date of filing: 15.05.2020
(51) Int. Cl.: C08B 30/04, C08H 99/00, C12N 9/42, C08H 8/00

(54) **METHOD FOR IMPROVING YIELD OF SPRAYED CORN BRAN IN CORN WEL-MILLING PROCESS**
VERFAHREN ZUR VERBESSERUNG DER AUSBEUTE AN VERSPRÜHTER MAISKLEIE IM MAISNASSMAHLVERFAHREN
PROCÉDÉ PERMETTANT D'AMÉLIORER LE RENDEMENT DE SON DE MAÏS PULVÉRISÉ DANS UN PROCÉDÉ DE BROYAGE DE MAÏS

(30) Priority: 15.05.2019 WO PCT/CN2019/087052
(43) Date of publication of application: 23.03.2022
(73) Proprietor: Novozymes A/S, 2880 Bagsværd (DK)
(72) Inventor: LIANG, Yongzhong, Beijing 100085 (CN); ZHANG, Yu, Beijing 100085 (CN); HAO, Jianming, Beijing 100085 (CN); CAO, Yi, Beijing 100085 (CN)
(74) Representative: NZ EPO Representatives
(86) International application number: PCT/CN2020/090394
(87) International publication number: WO 2020/228802

(56) References cited:
- WO-A1-2019/023222
- CN-A- 105 249 506
- CN-A- 106 832 009
- CN-A- 108 850 548
- CN-A- 109 688 843
- CN-U- 203 841 064
- JP-A- S62 244 318

## Description

### Technical Field

The present invention relates to a method for comprehensive utilization of by-products in a corn wet milling process, in particular to a method for increasing the yield of sprayed corn bran in a corn wet milling process.

### Background Art

Corn kernels contain starch, germ, fibers, proteins, and other substances that can be separated to prepare useful products. Typically, a wet milling method for wet milling corn kernels comprises the following steps: first impregnating the kernels with sulfurous acid for about 30 minutes to about 48 hours to start breaking starch and protein bonds, and the next step of the method involves coarse grinding to break the kernel skin and separate the germ from the remaining kernels. The remaining slurry composed of fibers, starch and proteins is finely ground and screened to separate the fibers from the starch and proteins, the starch is separated from the remaining slurry in a hydrocyclone, and the starch can then be converted into a syrup or alcohol, or dried and sold as corn starch, or modified by means of a chemical or physical method to produce modified corn starch. The fibers can be squeezed and dried as an animal feed.

As a by-product, the impregnation liquid that impregnates the corn kernels with sulfurous acid is a corn steep liquor, which is rich in soluble proteins, auxin and some precursor substances, tastes slightly salty, and is a commonly used organic nitrogen source for the growth of microorganisms. In an existing process, when a corn steep liquor is concentrated and mixed with fibers to produce high-protein feed sprayed corn bran, the sprayed amount of the corn steep liquor on the corn fibers is relatively small due to factors such as the color of the prepared sprayed corn bran being too dark. In recent years, the production of corn starch has been growing rapidly, especially in the production areas where corn starch factories are relatively concentrated, so that the outlet of corn steep liquor has become a problem, leading to large stocks of corn steep liquor in corn starch manufacturers or even discarding, which has brought serious environmental pressures, so this is an urgent problem that corn starch manufacturers need to solve.

### Summary of the Invention

The inventors of the present application have found that by adding an enzyme preparation during the process of separating fibers from starch and proteins in a corn wet milling process, the residual content of starch and/or proteins in the corn fiber residue by-product is reduced, thereby reducing the content of reducing sugars in the fiber residue, so that the color of finished sprayed corn bran can be controlled. In addition, the fiber residue after treatment with the enzyme preparation can have a lower water content and a looser and more bulky fiber residue structure. Therefore, while ensuring the normal color of the finished sprayed corn bran, this method can significantly increase the yield of the sprayed corn bran by-product in the corn wet milling process, i.e. significantly increase the sprayed amount of the concentrated corn impregnation liquid on the fiber corn bran, so that the problem of a large inventory amount of the corn steep liquor by-product during the corn starch production process can be alleviated, and the product value of the corn steep liquor by-product from the production of corn starch can be improved.

On this basis, the present invention relates to a method for increasing the yield of sprayed corn bran in a corn wet milling process, the method comprising the following steps:
a) impregnating corn kernels with a solution containing sulfurous acid, and collecting a corn impregnation liquid for concentration;
b) grinding the impregnated corn kernels to separate fibers from starch and proteins in the corn kernels, and collecting a fiber residue; and
c) mixing the concentrated corn impregnation liquid with the fiber residue such that the fiber residue adsorbs the concentrated corn impregnation liquid, and drying the mixture to prepare sprayed corn bran,
wherein in step b), an enzyme preparation is added during the separation of the fibers from the starch and proteins.

In a preferred embodiment of the present invention, the process of separating the fibers from the starch and proteins comprises a fiber washing step, and the fiber washing is carried out in the presence of the enzyme preparation.

In a preferred embodiment of the present invention, the enzyme preparation contains cellulase and/or hemicellulase, for example, cellulase, xylanase and/or arabinofuranosidase.

In a preferred embodiment of the present invention, the enzyme preparation contains cellulase, xylanase and arabinofuranosidase; preferably, the cellulase is expressed by *Trichoderma reesei;* preferably, the xylanase is GH10 xylanase; and preferably, the arabinofuranosidase is GH62 arabinofuranosidase.

### Detailed Description of Embodiments

The present invention relates to a method for increasing the yield of sprayed corn bran in a corn wet milling process, the method comprising the following steps:
a) impregnating corn kernels with a solution containing sulfurous acid, and collecting a corn impregnation liquid for concentration;
b) grinding the impregnated corn kernels to separate fibers from starch and proteins in the corn kernels, and collecting a fiber residue; and
c) mixing the concentrated corn impregnation liquid with the fiber residue such that the corn fiber residue adsorbs the concentrated corn impregnation liquid, and drying the mixture to prepare sprayed corn bran,
wherein in step b), an enzyme preparation is added during the separation of the fibers from the starch and proteins.

In the present invention, the corn is, for example, dent corn, flint corn, pod corn, striped corn, sweet corn, or waxy corn.

The wet milling process can vary significantly depending on the employed specific milling device. Preferably, the corn wet milling process is comprised of the following four steps: impregnation and germ separation, fiber washing and drying, starch and protein separation, and starch washing.

The purpose of impregnation is to soften the corn kernels, weaken the association between various components in the corn kernels, destroy the protein network in embryonic cells, remove most soluble substances, and separate a starch part from a non-starch part in the corn kernels to make the operation of the process easy. The impregnation process mainly involves a corn softening process and a diffusion process of soluble substances in corn, accompanied by a natural fermentation and action process of lactic acid bacteria.

Preferably, the impregnation of the corn kernels is carried out countercurrently in an aqueous sulfurous acid solution. Generally, a semi-continuous process is used, with 8 to 12 soaking tanks. During the soaking process, the corn kernels remain static in the tanks, and the soaking liquid is circulated itself in a tank while being transported to the previous stage of tank by means of a pump, such that a fresh sulfurous acid solution is always maintained in contact with the corn kernels experiencing the longest impregnation time (about to the end of soaking), while the corn kernels newly put into the tank are in contact with the impregnation liquid that is about to be discharged, so as to maintain the optimum impregnation effect. Preferably, the impregnation temperature is (50 ± 2)°C, the mass concentration of sulfurous acid during impregnation is 0.12-0.25%, and the impregnation time is 24-72 h, for example, 36-60 h. The soaking liquid that has completed the impregnation, i.e., a thin corn steep liquor that contains dry matter at a concentration of 6-9% by mass, is subjected to an evaporation process, for example, by a multi-effect falling film evaporation system, and a concentrated corn impregnation liquid with a dry matter concentration of about 40% by mass.

In a preferred embodiment of the present invention, during the impregnation process, the corn kernels absorb water, thereby increasing the moisture content thereof from 15% to 45% and making the size more than doubled. Optionally, for example, 0.12% sulfur dioxide (SO₂) and/or NaHSOs are added to the water to prevent bacteria from growing in a warm environment. As the corn expands and softens, the mild acidity of the impregnation water begins to loosen gluten bonds in the corn and release starch.

In a preferred embodiment of the present invention, after coarse grinding, the germ is separated by means of a cyclone based on the different physical properties of the corn endosperm slurry and the germ in terms of specific gravity; the corn endosperm slurry is then ground by means of a needle mill to free most of the starch and protein particles; then, with the aid of a sieving washing process, a crude starch milk containing starch and proteins is separated from fibers to obtain the crude starch milk with the fibers removed; according to the different physical properties of starch milk and gluten therein in terms of specific gravity, the starch milk and insoluble proteins are separated by means of a disc centrifuge; the starch milk obtained by centrifugation is then washed by means of a twelve-stage cyclone to remove residual gluten and solubles, so that a finished starch milk is obtained; and the finished starch milk is mechanically dehydrated by means of a centrifuge, and then dried, cooled and sieved to obtain commercial dry starch, while the gluten obtained by centrifugation is concentrated, dehydrated and dried to obtain a commercial protein powder product.

Preferably, the process of separating the fibers from the starch and proteins contains a fiber washing step, wherein during the fiber washing process, fine milling and screening are carried out to separate the fibers from the starch and proteins, preferably by using a multiple countercurrent washing method.

According to the difficulty of material washing and the actual requirements of production, a six-stage or seven-stage countercurrent washing method, i.e., six/seven instances of washing and six/seven instances of sieving, is used for the fiber residue. The sieving of the fiber residue is carried out using a curved sieve. The curved sieve is a curved sieve surface with many slender screen slits. The material to be separated is introduced into the upper portion of the curved sieve surface along the tangent line, and the material moves downward along the screen surface. It is at right angles to the screen slits. When the material enters the screen surface, the starch milk is scraped into the screen slits and falls down, so as to be separated from the fiber residue, and the starch milk is discharged from a lower collection pipeline. The oversize slides down to the lower portion of the screen surface under the action of gravity and is discharged.

Preferably, the thin slurry after the germ has been separated by means of a secondary cyclone separator is passed through a pressure curved sieve to obtain a coarse starch milk as the undersize, and the starch milk is combined with the coarse starch slurry separated after fine grinding and then enters a starch separation process; the oversize enters a needle mill for fine grinding to maximize the freeing of the starch from being bound with the fibers, and the finely ground slurry enters a fiber washing tank, where it is pumped to a first-stage pressure curved sieve together with washing water that washes the fibers, whereby a coarse starch milk is separated under the sieve, while the oversize is then washed countercurrently by means of a 7-stage pressure curved sieve, wherein washing process water is added in front of the last stage of sieve, passes through the sieve surface, moves forward stage by stage with the washed free starch, merges with the finely ground slurry, and enters the first-stage pressure curved sieve together, so as to separate a coarse starch milk, which merges with the coarse starch milk sieved before fine grinding and enters the starch separation process; and the fibers on the sieve surface are discharged from the last-stage curved sieve surface, then dehydrated by means of a screw extruder and subjected to a fiber feed process.

In a preferred embodiment of the present invention, the fiber washing step in the process of separating the fibers from the starch and proteins is carried out in the presence of the enzyme preparation.

In a preferred embodiment of the present invention, the process of separating the fibers from the starch and proteins comprises a fiber washing step, and the enzyme preparation can be added after fiber washing and before fiber dehydration. After fiber washing, the wet fiber residue is subjected to an enzyme treatment to further reduce the residual content of starch and/or proteins in the fiber residue by-product, so that a lower water content and a looser and more bulky fiber residue structure can be better formed.

In a preferred embodiment of the present invention, the enzyme preparation contains cellulase and/or hemicellulase, for example, cellulase, xylanase and/or arabinofuranosidase.

In a preferred embodiment of the present invention, the enzyme preparation contains cellulase, xylanase and arabinofuranosidase, preferably contains cellulase expressed by *Trichoderma reesei,* and GH10 xylanase and GH62 arabinofuranosidase.

"Cellulase" means one or more (e.g., several) enzymes that hydrolyze a cellulosic material. Two basic methods for measuring the activity of cellulolytic enzymes include: (1) measuring the activity of total cellulolytic enzymes, and (2) measuring the activity of an individual cellulolytic enzyme (endoglucanase, cellobiohydrolase, and β-glucosidase), as described in Zhang et al., 2006, Biotechnology Advances 24: 452-481. Insoluble substrates, including Whatman No. 1 filter paper, microcrystalline cellulose, bacterial cellulose, algae cellulose, cotton, pre-treated lignocellulose, etc., can be used to measure the activity of total cellulolytic enzymes. The most common method for measuring the total cellulose decomposing activity is a filter paper determination method using Whatman No. 1 filter paper as a substrate. The determination method is established by the International Union of Pure and Applied Chemistry (IUPAC) (Ghose, 1987, Pure Appl. Chem. 59: 257-68).

In a preferred embodiment of the present invention, the cellulase is expressed in an organism in the context of cellulase, e.g., *Trichoderma reesei.* The organism in the context of cellulase should be understood as an organism that naturally expresses one or more cellulolytic enzymes.

The term "xylanase" means 1,4-β-D-xylan-xylose hydrolase (E.C. 3.2.1.8), which catalyzes the endohydrolysis of 1,4-β-D-xylosidic bonds in xylan. The xylanase activity can be determined using 0.2% AZCL-arabinoxylan as a substrate in 0.01% TRITON^{®} X-100 and 200 mM sodium phosphate (pH 6) at 37°C. One unit of xylanase activity is defined as the production of 1.0 micromole of azurin per minute from 0.2% AZCL-arabinoxylan as a substrate in 200 mM sodium phosphate (pH 6) at 37°C and pH 6.

GH10 xylanase means that the xylanase is a member classified into the glycoside hydrolase family 10 in the database of carbohydrate active enzymes (CAZymes) available at http://www.cazy.org/.

In a preferred embodiment of the present invention, the xylanase has at least 80%, for example, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to a mature polypeptide of SEQ ID NO: 1. Preferably, the mature polypeptide of SEQ ID NO: 1 is amino acids 20 to 319 of SEQ ID NO: 1, and amino acids 1 to 19 of SEQ ID NO: 1 are a signal peptide sequence.

The term "arabinofuranosidase" means an α-L-arabinofuranoside arabinofuranohydrolase (EC 3.2.1.55), which catalyzes the hydrolysis of terminal non-reducing α-L-arabinofuranoside residues in α-L-arabinosides. The enzyme acts on α-L-arabinofuranosides, α-L-arabinans containing (1,3)- and/or (1,2)- and/or (1,5)-linkages, arabinoxylans and arabinogalactans. α-L-Arabinofuranosidase is also referred to as arabinosidase, α-arabinosidase, α-L-arabinosidase, α-arabinofuranosidase, polysaccharide α-L-arabinofuranosidase, α-L-arabinofuranoside hydrolase, L-arabinosidase, or α-L-arabinofuranosidase. 5 mg of medium-viscosity wheat arabinoxylan (Megazyme International Ireland, Ltd., Bray, Co., County Wicklow, Ireland) per ml of 100 mM sodium acetate (pH 5) can be used in a total volume of 200 µl at 40°C for 30 minutes, followed by arabinose analysis by AMINEX^{®} HPX-87H column chromatography (Bio-Rad Laboratories, Inc., Hercules, California, USA) to determine the arabinofuranosidase activity.

GH62 arabinofuranosidase means that the arabinofuranosidase is a member classified into the glycoside hydrolase family 62 in the database of carbohydrate active enzymes (CAZymes).

In a preferred embodiment of the present invention, the arabinofuranosidase has at least 80%, for example, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to a mature polypeptide of SEQ ID NO: 2. Preferably, the mature polypeptide of SEQ ID NO: 2 is amino acids 27 to 332 of SEQ ID NO: 2, and amino acids 1 to 26 of SEQ ID NO: 2 are a signal peptide sequence.

The degree of association between two amino acid sequences or between two nucleotide sequences is described by the parameter "sequence identity".

For the purpose of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. Version 6.1.0 is used. The optional parameters used are gap open penalty of 10, gap extension penalty of 0.5, and the EBLOSUM62 (EMBOSS version of BLOSUM62) substitution matrix. The output of Needle labeled "longest identity" (obtained using the - nobrief option) is used as the percent identity and is calculated as follows: (Identical Residues x 100)/(Length of Alignment - Total Number of Gaps in Alignment).

The cellulase, arabinofuranosidase or xylanase of the present invention can be obtained from microorganisms of any genus. For the purpose of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

Preferably, in one embodiment, the arabinofuranosidase of the present invention is obtained from *Aspergillus niger.*

Preferably, in one embodiment, the xylanase of the present invention is obtained from *Aspergillus niger.*

In a preferred embodiment of the present invention, when separating the fibers from the starch and proteins, the reaction time of the enzyme preparation acting on the fibers containing the starch and proteins is at least 10 minutes or more, for example, at least 15 minutes or at least 30 minutes, preferably at least 45 minutes.

In a preferred embodiment of the present invention, the residual content of the starch (on a dry fiber basis) in the fiber residue is reduced by at least 2% by weight, for example, by at least 3%, or by at least 4%, preferably by at least 5%, compared with a process without the enzyme treatment.

In a preferred embodiment of the present invention, the residual content of the proteins (on a dry fiber basis) in the fiber residue is reduced by at least 0.5% by weight, for example, by at least 1%, preferably by at least 1.5%, compared with a process without the enzyme treatment.

In a preferred embodiment of the present invention, the residual content of water in the fiber residue is reduced by at least 2% by weight, for example, by at least 5%, preferably by at least 6%, compared with a process without the enzyme treatment.

In a preferred embodiment of the present invention, the fiber residue after treatment with the enzyme preparation can have a looser and more bulky structure.

In a preferred embodiment of the present invention, the fiber residue and the concentrated corn steep liquor are generally dried using a tube-bundle drying device after being mixed in the present invention, and the drying temperature is preferably 80-100°C. The tube-bundle dryer is a contact (indirect) dryer, and the structure thereof is a transmissionable heating tube bundle with a shovel built in a fixed housing. A transmission system drives the tube bundle means to rotate. Steam enters a tube through a rotary joint and transfers heat to become condensed water to be discharged; The moisture in the material is heated outside the tube to become steam and is discharged by means of a dehumidifier fan, the material is lifted in the tube bundle by the driven shovel, evenly falls to the outer surface of the heating tube, and is pushed from an inlet end to an outlet by the shovel, whereupon the material is dried.

In a preferred embodiment of the present invention, the process control parameters of the fiber drying process are: a steam pressure of 0.4-0.6 MPa, a moisture content of less than 12% after drying, a steam temperature of 150-210°C, and a fiber tube bundle exhaust temperature 80-98°C.

In a preferred embodiment of the present invention, before step c), at least part of the fiber residue is dried, or is mixed with some dry fiber residue.

In a preferred embodiment of the present invention, after the sprayed corn bran is prepared for the first time, the sprayed corn bran can be further mixed with the concentrated corn impregnation liquid to further adsorb the concentrated corn impregnation liquid, and the sprayed corn bran is dried to produce sprayed corn bran with a higher sprayed amount of corn steep liquor.

In a preferred embodiment of the present invention, at least part of the fiber residue is brought into the tube-bundle dryer for preliminary drying, the fiber residue after preliminary drying is sent to a blender by means of a screw conveyor (or to this screw conveyor), mixed with the concentrated corn impregnation liquid, and then brought into the next stage of tube-bundle dryer for further drying until the water content of the sprayed corn bran is 8-12% (w/w), thereby producing a sprayed corn bran product.

In a preferred embodiment of the present invention, the water content of the fiber residue after dehydration by an extruder is controlled to be ≤ 65% (w/w), the fiber residue is then brought into a screw conveyor, and mixed with the concentrated corn impregnation liquid at a certain ratio in the screw conveyor, and the mixture is sent to a tube-bundle dryer and dried until the water content of the sprayed corn bran is 8-12% (w/w), thereby producing a sprayed corn bran product.

In a preferred embodiment of the present invention, the produced sprayed corn bran product is powdery or granular, free of molds, agglomeration, and moth-eaten appearance, and light yellow or brown, and has a protein content that is ≥ 18% (w/w), for example, can reach at least 19% (w/w), at least 20% (w/w), at least 21% (w/w), or at least about 22% (w/w), and a water content of 8-12% (w/w), and the produced sprayed corn bran can be added to an animal feed at a ratio of 3-5% (w/w).

In the present invention, by adding an enzyme preparation during the process of separating fibers from starch and proteins in a corn wet milling process of corn kernels, the residual content of starch and/or proteins in the corn fiber residue by-product is reduced, thereby reducing the content of reducing sugars in the fiber residue, so that the color of finished sprayed corn bran can be controlled. In addition, the fiber residue after treatment with the enzyme preparation can have a lower water content and a looser and more bulky fiber residue structure. Therefore, while ensuring the normal color of the finished sprayed corn bran, this method can significantly increase the yield of the sprayed corn bran in the corn wet milling process, i.e. significantly increase the sprayed amount of the concentrated corn impregnation liquid on the fiber corn bran, so that the problem of a large inventory amount of the corn steep liquor by-product during the corn starch production process can be alleviated, and the product value of the corn steep liquor by-product from the production of corn starch can be improved.

The invention described and claimed herein is not limited to the scope of the specific embodiments disclosed herein, and these embodiments are only intended to illustrate several aspects of the present invention.

### Materials and Methods

Cellulase: Celluclast 1.5 L (available from Novozymes), expressed by *Trichoderma reesei.*

GH10 xylanase: GH10 xylanase derived from *Aspergillus niger* (SEQ ID NO: 1).

GH62 arabinofuranosidase: GH62 arabinofuranosidase derived from *Aspergillus niger* (SEQ ID NO: 2).

### Assessment method:

Determination of residual starch content: 2.5 grams of a dried fiber residue sample was accurately weighed, and 1.124% (w/w) hydrochloric acid was added for a reaction in a boiling water bath to convert starch in the sample into glucose, which had a property of rotating polarized light. A polarimeter was then used to measure the optical rotation of the hydrolysate, and the starch content of the sample was then calculated according to an equation.

Determination of residual protein content: 3 grams of a dried fiber residue sample was accurately weighed into a Kjeldahl flask, concentrated sulfuric acid was added, and the mixture was boiled for decomposition. After the decomposed liquid was cooled, 40% (w/w) sodium hydroxide was added to neutralize the decomposed liquid, and distillation was carried out to release ammonia. The released ammonia was collected with 2% (w/w) boric acid and titrated with a 0.05 mol/L sulfuric acid solution to determine the content of nitrogen in the sample, and the protein content of the sample was then calculated according to an equation.

Determination of water content: 5 grams of a fiber residue sample was accurately weighed in a constant weight aluminum box, the constant weight aluminum box was placed in an oven at 131°C, dried for 40 minutes, taken out, and quickly capped. It was placed in a dryer and cooled to room temperature, the sample after drying was weighed, and the water content of the sample was then calculated according to an equation.

Determination of the color of the sprayed corn bran: 5 experienced engineers were selected to evaluate the color of the produced sprayed corn bran, and classified it according to light yellow, brownish yellow and tannish yellow. A lighter color indicated that the quality of the produced sprayed corn bran was better.

### Example

A corn wet milling production system that processed 1,000 tons of a corn kernel raw material per day (the water content of corn kernels was 14% W/W) was taken as an example:
Cleaned corn kernels were first placed in a sulfurous acid solution with a concentration of 0.12% (w/w) at a temperature of 50°C and impregnated for 48 hours, wherein the mass ratio of the corn kernels to the aqueous sulfurous acid solution was 0.8 : 1; the impregnated and softened corn kernels were coarsely ground, the germ was separated by means of a cyclone based on the different physical properties of the corn endosperm slurry and the germ in terms of specific gravity; the corn endosperm slurry was then ground by means of a needle mill to free most of the starch and protein particles; then, with the aid of a sieving washing process, a crude starch milk containing starch and proteins was separated from fibers to obtain the crude starch milk with the fibers removed; according to the different physical properties of starch milk and gluten therein in terms of specific gravity, the starch milk and insoluble proteins were separated by means of a disc centrifuge; the starch milk obtained by centrifugation was then washed by means of a twelve-stage cyclone to remove residual gluten and solubles, so that a finished starch milk was obtained; and the finished starch milk was mechanically dehydrated by means of a centrifuge, and then dried, cooled and sieved to obtain commercial dry starch, while the gluten obtained by centrifugation was concentrated, dehydrated and dried to obtain a commercial protein powder product.

The corn impregnation liquid by-product, which was dilute corn steep liquor with a dry matter concentration of about 9% (w/w), was collected, subjected to an evaporation process and concentrated into a corn steep liquor with a dry matter concentration of about 40% (w/w), i.e., concentrated corn impregnation liquid.

The process of separating the fibers from the starch and proteins contained a fiber washing step in which a seven-stage countercurrent washing method was used to separate a crude starch milk containing the starch and proteins from the fibers, and fiber residue by-product A was collected, wherein the water content of the fiber residue was 60.2% by weight, the residual starch content of the fiber residue (on a dry fiber basis) was 17.8% by weight, and the protein content of the fiber residue (on a dry fiber basis) was 10.6% by weight.

For comparison, where all the other conditions remained unchanged, the inventors of the present application added an enzyme preparation containing cellulase, GH10 xylanase, GH62 arabinofuranosidase and GH10 xylan (the addition ratio of the cellulase, GH10 xylanase and GH62 arabinofuranosidase was about 80 : 15 : 5 by weight) during the process of separating the fibers from the starch and proteins, so that the fiber washing step was carried out in the presence of the enzyme preparation, wherein the addition amount of the enzyme preparation was 1.125 kg/ton corn on a dry basis, the enzyme reaction time under the conditions of pH 4.0 and a temperature of 50°C was about 15 minutes, and fiber residue by-product B was collected, wherein the water content of the fiber residue was 54.0% by weight, the residual starch content of the fiber residue (on a dry fiber basis) was 12.6% by weight, and the residual protein content of the fiber residue (on a dry fiber basis) was 9.2% by weight. Compared with fiber residue A, fiber residue B had a looser and more bulky structure.

The collected fiber residue by-product A and the collected fiber residue by-product B were respectively placed in a tube-bundle dryer at a temperature of 90°C for 30 minutes, the collected by-product concentrated corn impregnation liquid was then evenly sprayed onto the pre-dried fiber residue by means of a sprayer, the mixture was mixed in a conveying auger, the mixed sprayed corn bran was heated and dried at 90°C in a tube-bundle dryer, and when the color of the produced sprayed corn bran became brownish yellow, drying was stopped to produce the product.

The color of the sprayed corn bran product made from fiber residue by-product A as a raw material was brown. Taking a corn wet mill production system that processed 1,000 tons of corn kernels per day as an example, in the case of making full use of the fiber residue by-product that could be obtained by means of the corn wet milling production system, the output of the sprayed corn bran product that could be prepared thereby was 136.2 tons.

In contrast, when sprayed corn bran was prepared from fiber residue by-product B as a raw material, taking a corn wet mill production system that processed 1,000 tons of corn kernels per day as an example, in the case of making full use of the fiber residue by-product that could be obtained by means of the corn wet milling production system, the color of the prepared sprayed corn bran was close to brownish yellow and the color lay in the range between light yellow and brownish yellow, and the output of the sprayed corn bran product prepared thereby had reached 142.7 tons. According to the color of the sprayed corn bran product, it was indicated that not only did the sprayed corn bran made from fiber residue B under these conditions have a higher yield, but also the quality was better than the sprayed corn bran made of fiber residue A.

This indicates that in the process of the present invention, since the fiber washing step of the corn wet milling process is carried out in the presence of an enzyme preparation containing cellulase, GH62 arabinofuranosidase and GH10 xylanase, the corn fiber residue resulting therefrom has a lower water content, a lower residual starch content, a lower residual protein content, and a more suitable fiber residue structure, and therefore, where it is ensured that the color of the produced sprayed corn bran has a high quality (for example, a lighter product color), more concentrated corn impregnation liquid can be adsorbed, so that where the corn kernel raw material processing amount is the same, the output of the produced sprayed corn bran can be significantly increased, and the commercial value of the fiber residue by-product, especially the low-value corn impregnation liquid by-product, is improved.

## Claims

1. A method for increasing the yield of sprayed corn bran in a corn wet milling process, the method comprising the following steps:
a) impregnating corn kernels with a solution containing sulfurous acid, and collecting a corn impregnation liquid for concentration;
b) grinding the impregnated corn kernels to separate fibers from starch and proteins in the corn kernels, and collecting a fiber residue; and
c) mixing the concentrated corn impregnation liquid with the fiber residue such that the fiber residue adsorbs the concentrated corn impregnation liquid, and drying the mixture to prepare sprayed corn bran,
wherein in step b), an enzyme preparation is added during the separation of the fibers from the starch and proteins.

2. The method of claim 1, wherein the process of separating the fibers from the starch and proteins comprises a fiber washing step, and the fiber washing is carried out in the presence of the enzyme preparation.

3. The method of claim 1, wherein the process of separating the fibers from the starch and proteins comprises a fiber washing step, and the enzyme preparation is added after fiber washing and before fiber dehydration.

4. The method of claim 1, wherein the enzyme preparation contains cellulase and/or hemicellulase, for example, cellulase, xylanase and/or arabinofuranosidase.

5. The method of claim 1, wherein the enzyme preparation contains cellulase, xylanase and arabinofuranosidase; preferably, the cellulase is expressed by *Trichoderma reesei;* preferably, the xylanase is GH10 xylanase; and preferably, the arabinofuranosidase is GH62 arabinofuranosidase.

6. The method of claim 1, wherein when separating the fibers from the starch and proteins, the reaction time of the enzyme preparation acting on the fibers containing the starch and proteins is at least 10 minutes or more, for example, at least 15 minutes or at least 30 minutes, preferably at least 45 minutes.

7. The method of claim 1, wherein the residual content of the starch (on a dry fiber basis) in the fiber residue is reduced by at least 2% by weight, for example, by at least 3%, or by at least 4%, preferably by at least 5%, compared with a process without the enzyme treatment.

8. The method of claim 1, wherein the residual content of the proteins (on a dry fiber basis) in the corn fiber residue is reduced by at least 0.5% by weight, for example, by at least 1%, preferably by at least 1.5%, compared with a process without the enzyme treatment.

9. The method of claim 1, wherein before step c), at least part of the fiber residue is dried, or is mixed with some dry fiber residue.

10. The method of claim 1, wherein after the sprayed corn bran is prepared for the first time, the sprayed corn bran can be further mixed with the concentrated corn impregnation liquid to further adsorb the concentrated corn impregnation liquid.

11. The method of claim 1, wherein the drying in step c) is carried out in a tube-bundle drying device, preferably at a drying temperature of 80-100°C.

## Patentansprüche

1. Verfahren zum Erhöhen der Ausbeute an besprühter Maiskleie bei einem Mais-Nassmahlverfahren, wobei das Verfahren die folgenden Schritte umfasst:
a) Imprägnieren von Maiskernen mit einer Lösung, die Schwefelsäure enthält, und Sammeln einer Mais-Imprägnierflüssigkeit zum Konzentrieren;
b) Mahlen der imprägnierten Maiskerne, um Fasern von Stärke und Proteinen in den Maiskernen abzutrennen, und Sammeln eines Faserrückstands; und
c) Mischen der konzentrierten Mais-Imprägnierflüssigkeit mit dem Faserrückstand, so dass der Faserrückstand die konzentrierte Mais-Imprägnierflüssigkeit adsorbiert, und Trocknen des Gemischs, um besprühte Maiskleie zu erhalten,
wobei bei Schritt b) eine Enzymzubereitung während des Abtrennens der Fasern von der Stärke und den Proteinen zugegeben wird.

2. Verfahren gemäß Anspruch 1, wobei das Verfahren des Abtrennens der Fasern von der Stärke und den Proteinen einen Faserwaschschritt umfasst und das Faserwaschen in Gegenwart der Enzymzubereitung durchgeführt wird.

3. Verfahren gemäß Anspruch 1, wobei das Verfahren des Abtrennens der Fasern von der Stärke und den Proteinen einen Faserwaschschritt umfasst und die Enzymzubereitung nach dem Faserwaschen und vor Faserdehydratation zugegeben wird.

4. Verfahren gemäß Anspruch 1, wobei die Enzymzubereitung Cellulase und/oder Hemicellulase, beispielsweise Cellulase, Xylanase und/oder Arabinofuranosidase, enthält.

5. Verfahren gemäß Anspruch 1, wobei die Enzymzubereitung Cellulase, Xylanase und Arabinofuranosidase enthält; vorzugsweise die Cellulase von *Trichoderma reesei* exprimiert wird; vorzugsweise die Xylanase GH10-Xylanase ist; und vorzugsweise die Arabinofuranosidase GH62-Arabinofuranosidase ist.

6. Verfahren gemäß Anspruch 1, wobei bei dem Abtrennen der Fasern von der Stärke und den Proteinen die Reaktionszeit des Einwirkens der Enzymzubereitung auf die Fasern, die die Stärke und Proteine enthalten, wenigstens 10 Minuten oder mehr, beispielsweise wenigstens 15 Minuten oder wenigstens 30 Minuten, vorzugsweise wenigstens 45 Minuten, beträgt.

7. Verfahren gemäß Anspruch 1, wobei der Restgehalt an der Stärke (auf einer Trockenfaserbasis) in dem Faserrückstand um wenigstens 2 Gew.-%, beispielsweise um wenigstens 3 % oder um wenigstens 4 %, vorzugsweise um wenigstens 5 %, im Vergleich zu einem Verfahren ohne die Enzymbehandlung verringert ist.

8. Verfahren gemäß Anspruch 1, wobei der Restgehalt an den Proteinen (auf einer Trockenfaserbasis) in dem Maisfaserrückstand um wenigstens 0,5 Gew.-%, beispielsweise um wenigstens 1 %, vorzugsweise um wenigstens 1,5 %, im Vergleich zu einem Verfahren ohne die Enzymbehandlung verringert ist.

9. Verfahren gemäß Anspruch 1, wobei vor Schritt c) wenigstens ein Teil des Faserrests getrocknet wird oder mit etwas an trockenem Faserrest gemischt wird.

10. Verfahren gemäß Anspruch 1, wobei nach der erstmaligen Herstellung der besprühten Maiskleie die besprühte Maiskleie ferner mit der konzentrierten Mais-Imprägnierflüssigkeit gemischt werden kann, um die konzentrierte Mais-Imprägnierflüssigkeit weiter zu adsorbieren.

11. Verfahren gemäß Anspruch 1, wobei das Trocknen bei Schritt c) in einer Rohrbündel-Trocknungsvorrichtung durchgeführt wird, vorzugsweise bei einer Trocknungstemperatur von 80-100 °C.

## Revendications

1. Procédé pour augmenter le rendement de son de maïs pulvérisé dans un procédé de mouture humide de maïs, le procédé comprenant les étapes suivantes :
a) imprégnation de grains de maïs avec une solution contenant de l'acide sulfureux, et collecte d'un liquide d'imprégnation de maïs pour une concentration ;
b) broyage des grains de maïs imprégnés pour séparer des fibres de l'amidon et de protéines dans les grains de maïs, et collecte d'un résidu de fibres ; et
c) mélange du liquide d'imprégnation de maïs concentré avec le résidu de fibres de sorte que le résidu de fibres adsorbe le liquide imprégnation de maïs concentré, et séchage du mélange pour préparer du son de maïs pulvérisé,
dans l'étape b), une préparation enzymatique étant ajoutée pendant la séparation des fibres de l'amidon et des protéines.

2. Procédé selon la revendication 1, le processus de séparation des fibres de l'amidon et des protéines comprenant une étape de lavage de fibres, et le lavage de fibres étant mis en œuvre en la présence de la préparation enzymatique.

3. Procédé selon la revendication 1, le processus de séparation des fibres de l'amidon et des protéines comprenant une étape de lavage de fibres, et la préparation enzymatique étant ajoutée après un lavage de fibres et avant une déshydratation de fibres.

4. Procédé selon la revendication 1, la préparation enzymatique contenant une cellulase et/ou une hémicellulase, par exemple, une cellulase, une xylanase et/ou une arabinofuranosidase.

5. Procédé selon la revendication 1, la préparation enzymatique contenant une cellulase, une xylanase et une arabinofuranosidase ; préférablement, la cellulase étant exprimée par *Trichoderma reesei ;* la xylanase étant la xylanase GH10 ; et préférablement, l'arabinofuranosidase étant l'arabinofuranosidase GH62.

6. Procédé selon la revendication 1, dans lequel lors de la séparation des fibres de l'amidon et des protéines, le temps de réaction de la préparation enzymatique agissant sur les fibres contenant l'amidon et les protéines est d'au moins 10 minutes ou plus, par exemple, d'au moins 15 minutes ou d'au moins 30 minutes, préférablement d'au moins 45 minutes.

7. Procédé selon la revendication 1, la teneur résiduelle de l'amidon (sur une base de fibres sèches) dans le résidu de fibres étant réduite d'au moins 2 % en poids, par exemple, d'au moins 3 %, ou d'au moins 4 %, préférablement d'au moins 5 %, par comparaison avec un processus sans le traitement enzymatique.

8. Procédé selon la revendication 1, la teneur résiduelle des protéines (sur une base de fibres sèches) dans le résidu de fibres de maïs étant réduite d'au moins 0,5 % en poids, par exemple, d'au moins 1 %, préférablement d'au moins 1,5 %, par comparaison avec un processus sans le traitement enzymatique.

9. Procédé selon la revendication 1, dans lequel avant l'étape c), au moins une partie du résidu de fibres est séchée, ou est mélangée avec une certaine quantité de résidu de fibres sèches.

10. Procédé selon la revendication 1, dans lequel après que le son de maïs pulvérisé a été préparé pour la première fois, le son de maïs pulvérisé peut être en outre mélangé avec le liquide d'imprégnation de maïs concentré pour adsorber en outre le liquide d'imprégnation de maïs concentré.

11. Procédé selon la revendication 1, le séchage dans l'étape c) étant mis en œuvre dans un dispositif de séchage à faisceau de tubes, préférablement à une température de séchage de 80 à 100 °C.
